# EUROPEAN PATENT APPLICATION

(11) **EP 2 216 058 A1**
(43) Date of publication of application: **11.08.2010**
(21) Application number: 08846761.8
(22) Date of filing: 07.11.2008
(51) Int. Cl.: A61M 1/00

(54) **SUCTION NOZZLE AND ASPIRATOR FOR REMOVING NASAL SECRETIONS FROM INFANTS USING A DEVICE THAT DOES NOT CAUSE CONTAMINATION OR TRANSMIT PATHOGENIC GERMS**

(30) Priority: 08.11.2007 ES 200702305 U
(71) Applicant: Italfarmaco, S.A., 28108 Alcobendas (Madrid) (ES)
(72) Inventor: COBO DE LA CRUZ, José, E-28003 Madrid (ES)
(74) Representative: ABG Patentes, S.L.
(86) International application number: PCT/ES2008/000692
(87) International publication number: WO 2009/060111

(57) **Abstract**

The invention relates to a suction nozzle of the nasal aspirator with a one-way air flow selection valve system for a nasal aspirator which comprises three parts perfectly assembled to one another: an outer case, an inner case and a flow selection valve membrane, the result of which is a single fixed body which houses a valve system therein and from which a suction cannula projects at its proximal end and a hollow body suitable for being coupled to a tube of a nasal aspirator projects at its distal end. A nasal aspirator comprising said suction nozzle is also contemplated.

## Description

### Technical Field

The present invention relates to a suction nozzle and a nasal aspirator, designed to aspirate the secretions which accumulate in the nasal passages with maximum efficiency and safety, including mechanisms that do not induce bacterial contamination.

The features of the nasal aspirator of the invention make it especially suitable for cleaning nasal secretions of infants and breast-fed infants, both in normal physiological situations and after pathological alterations of the mucosae and the corresponding organs, such as catarrhs, colds and allergic processes.

### Background of the Invention

It is known in the field of caring for infants, mainly breast-fed infants, that it is necessary to maintain their nasal passages clean and clear of secretions and mucus. This cleaning is usually performed by means of the suction of the nasal secretions, using a series of apparatuses and devices available on the market for such purpose. The suction process must be hygienic, effective and non-injuring for the patient (the infant).

Although the current methods for carrying out the nasal suction partially cover these needs, they still have a series of drawbacks:
- they do not prevent an additional nasal contamination by the person performing the suction (who will be referred to as "use" throughout the document) or by the utensil itself;
- they lack a graduation of the suction;
- they are not comfortable to use;
- they have an inherent traumatic effect (bulb, cannula); and/or
- they cause infant rejection.

### Description of the Invention

The object of the proposed invention is to solve the problems set forth above, favoring in the nasal suction process the efficiency, safety and ease of use and acceptability by both the patient and the user and eliminating the risk of contamination and transmission of pathogenic germs. A suction nozzle and a nasal aspirator, defined in independent claims 1 and 7, are presented for this purpose.

The suction nozzle and the nasal aspirator of the invention are designed to remove the nasal secretions of the infant from the choanae by means of the suction force exerted by the user (parents or caregiver) upon aspirating with the mouth. There is therefore a risk of inoculating germs from the adult's mouth to the respiratory system of the infant. The mouth, a septic area, can transmit any pathogenic germ (tuberculosis, meningitis, pneumonia or virus) and it is obvious that the induction of contamination or transmission of pathogenic germs should be prevented.

Under these premises, the present invention contemplates the principle of "first, do no harm" and consequently the non-induction of the possibility of transmission of bacteria or any pathogenic germ has been provided.

More specifically, in a first aspect the present invention relates to a suction nozzle with a valve system according to independent claim 1, to be coupled to a nasal suction apparatus.

The suction nozzle of the nasal aspirator of the invention comprises three parts perfectly assembled to one another: an outer case, an inner case and a flow selection valve membrane, the result of which is a single fixed body which houses a valve system therein and from which a suction cannula projects at its proximal end and a hollow element suitable for being coupled to a tube of a nasal aspirator projects at its distal end. Throughout this document, the terms "distal" and "proximal" must be interpreted taking the position of the user during the normal use of the suction nozzle and of the nasal aspirator of the invention as a reference.

The inner part of the suction nozzle furthermore has, in its inner part, at least one stop serving as a support for the bending of the valve membrane which, upon aspirating, bends over these points, bending against the suction force and allowing the air flow and the suction function. On the contrary, when air is blown or flows in the opposite direction, it pushes the membrane towards the air outlet and closes it hermetically, preventing the transmission of possible pathogenic germs from the user's mouth to the infant's nose.

The suction nozzle of the present invention therefore provides significant advantages for the patient and the person performing the suction which have not been contemplated in other nasal aspirators, apparatuses or instruments used today.

The suction nozzle with a valve system of the invention can have various designs and, therefore, manufacturing options, each of them with particular and specific features. Two possible embodiments, which will be described below, have been considered herein. The essential difference between these embodiments is the location of the support points of the bending of the valve membrane, which form part of the inner cylindrical case in the first embodiment and form part of the outer cylindrical case in the second embodiment.

Nevertheless, the functional concept of the valve system with a one-way air flow selection membrane is identical in both embodiments (and in other embodiments not explicitly described herein, but comprised in the scope of protection of the invention). In both embodiments, the valve function does not change either in quality or in intensity and the safety, efficiency, use comfort, non-induction of contamination and transmission of pathogenic germs are similar.

In a second aspect of the invention, a nasal aspirator according to independent claim 7, suitable for being coupled to and cooperating with a suction nozzle such as the one defined in claim 1, is provided.

Dependent claims 2-6 and 8-10 define the preferred embodiments of the present invention.

### Description of the Drawings

To complement the description which will be made below and for the purpose of aiding to better understand the features of the invention according to a preferred practical embodiment thereof, a set of drawings is attached as an integral part of said description, in which the following has been depicted with an illustrative and non-limiting character:
Figure 1 shows a sagittal section of a suction nozzle with a valve system according to a first embodiment of the invention.
Figure 2 shows a sagittal section of a suction nozzle with a valve system in a closed position in relation to the air flow.
Figure 3 shows a front section of a suction nozzle with a valve system in a closed position, preventing the air flow.
Figure 4 shows a sagittal section of a suction nozzle with a valve system in an open position in relation to the air flow.
Figure 5 shows a front section of a suction nozzle with a valve system in an open position, to be able to perform the suction.
Figure 6 shows a front section and a side section of an outer cylindrical case of a suction nozzle according to a second embodiment of the invention.
Figure 7 shows a side section of an outer cylindrical case of a suction nozzle according to a second preferred embodiment of the invention.
Figure 8 shows a front section and a side section of an inner cylindrical case of a suction nozzle according to a second embodiment of the invention.
Figure 9 shows a nasal aspirator according to the invention.

### Preferred Embodiment of the Invention

Two possible embodiments of the suction nozzle (1) with a valve system of the invention are described herein.

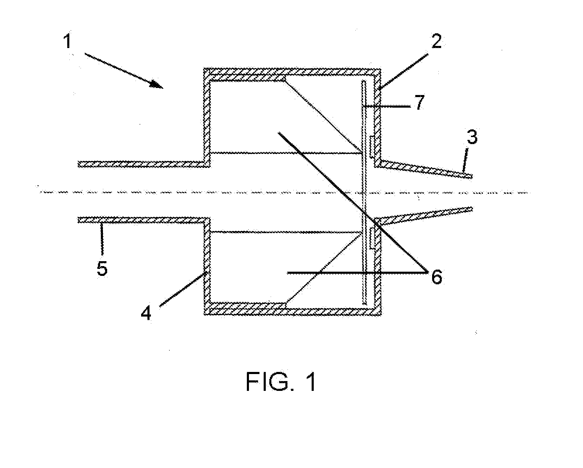

In the embodiment which is observed in Figures 1-5, the suction nozzle (1) with a valve system of the invention comprises an inner cylindrical case (4), the inner part of which has a support stop (6) formed by four flanges with an end part finished in a point and serving for the support of the flow selection valve membrane (7). On the proximal face of the inner cylindrical case (4) there projects, in the central part, a suction cannula (5) which is applied by the subject to his mouth to perform the suction.

The air flow selection valve membrane (7) is housed in the space between the inner distal face of the outer case (2) and the support stop (6); it is a flexible sheet of plastic material with a high elastic memory capacity, in one possible embodiment it is a sheet of polyolefin plastomer (for example a sheet of transparent plastic sold under the Affinity® trade mark).

An outer cylindrical case (2), forming the outer visible part of the suction nozzle (1), covers the entire valve system which is located therein, except the face where the suction cannula (5) is located, which corresponds with the outer face of the inner cylindrical case (4). On the opposite face, belonging to the outer cylindrical case (2), in its central area, there is a cylindrical hollow coupling body (3) suitable for being hermetically coupled to a tube of a nasal aspirator. The walls are smooth and polished, facilitating the holding of the suction nozzle (1) with a valve system.

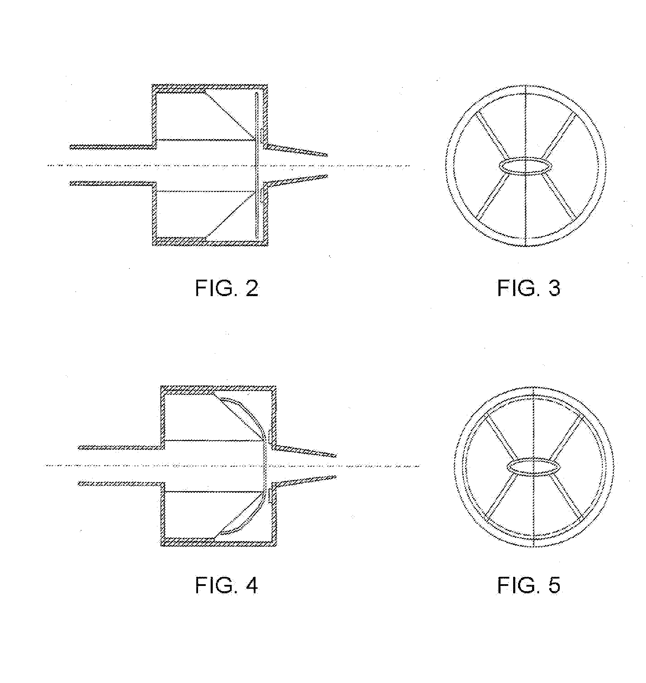

When the user sucks on the suction cannula (5), the valve membrane (7) bends over the support stop (6), adopting a configuration such as the one observed in Figures 4 and 5, allowing the passage of the air penetrating through the coupling body (3) and circulating through the circular crown delimited by the bent valve membrane (7) and the wall of the outer cylindrical case (2), thus allowing the suction. On the contrary, when the user blows on the suction cannula, the air pushes the membrane towards the hole of the coupling body (3), hermetically closing the air outlet, as observed in Figures 2 and 3, and preventing possible pathogenic germs of the user's mouth from reaching the infant's nose.

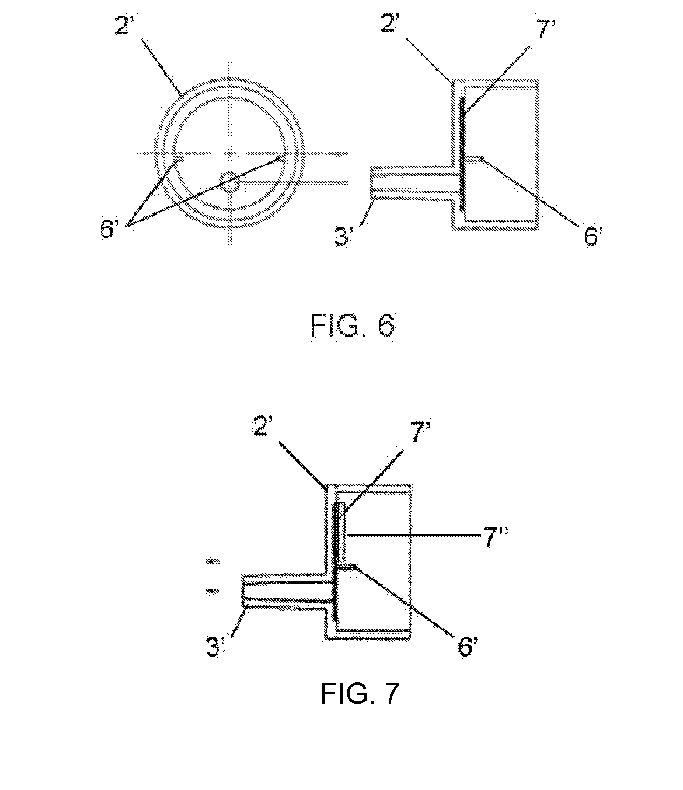

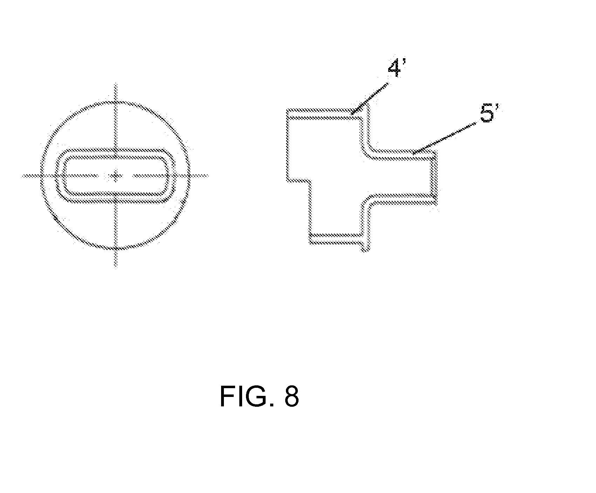

Figures 6, 7 and 8 show the elements of the suction nozzle (1) according to a second embodiment of the present invention.

In this embodiment, the outer cylindrical case (2') houses the valve membrane (7') and the support stops (6') for the bending of the mentioned membrane. Specifically, there are two support stops (6') projecting from the inner side wall in the bottom middle third of the outer cylindrical case (2'). These stops (6') are located facing one another, at the same height, slightly above the line of the inlet hole of the aspirated air and at a distance from the distal face to be able to house the valve membrane (7') between it and the support stops (6'), as can be seen in Figure 6.

In a preferred embodiment, the exact location of these two supports (6') is at 0.5 mm from the inner distal face of the outer case (2'), a space occupied by the valve membrane (7'); their length is 5 mm from their base (inner face of the outer cylindrical case (2)) to their final end and their thickness is 0.6 mm.

The air flow selection valve membrane (7') is a flexible circular sheet of plastic material with high elastic memory capacity, in one possible embodiment it is a sheet of elastomer.

In a preferred embodiment, this valve membrane has a semicircular reinforcement (7'') facilitating the bending of the membrane during the use of the nasal aspirator. Said reinforcement is adhered to the membrane, occupying the surface above the line of the support stops (6'), i.e., in the top half of the membrane. In one embodiment, the reinforcement is made of the same material as the membrane.

In this second embodiment, the cylindrical hollow coupling body (3') suitable for being hermetically coupled to a tube (12) of the nasal aspirator is located in the bottom third of the distal face of the outer cylindrical case (2').

The inner cylindrical case (4') does not have therein any support point for the bending of the valve membrane (7') in this embodiment and its inner walls are smooth and cylindrical, as seen in Figure 7, allowing an air flow with hardly any resistance. On the proximal face of the inner cylindrical case (4') there projects, in the central part, a suction cannula (5') and in the distal part the inner cylindrical case (4') is finished in the form of a step at the height of the two bending support stops (6') which are located in the outer cylindrical case (2'), facilitating that the entire part is fixed and embedded.

The operating principle is similar to the one described in the first embodiment: When the user sucks on the suction cannula (5'), the valve membrane (7') bends over the support stops (6') in the area where the suction occurs, i.e., on the bottom half of the membrane, allowing the passage of the air penetrating through the coupling body (3') and allowing the suction. On the contrary, when the air flow is in the opposite direction, it pushes the membrane towards the hole of the coupling body (3'), hermetically closing the air outlet.

The outer case (2,2') and inner case (4,4') of the suction nozzle of the invention can be manufactured in any material acceptable for sanitary use and suitable for the functions that it fulfills, in one embodiment it can be anti-shock (high-impact) polystyrene.

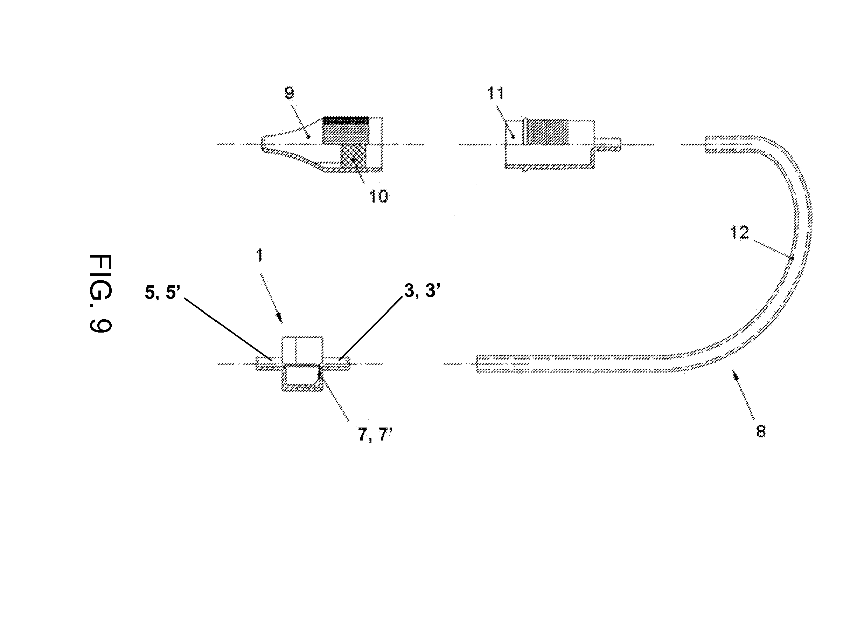

As has been mentioned above, the suction nozzle (1) is designed to be suitably coupled to a nasal aspirator (8) and cooperate therewith. Figure 9 shows a nasal aspirator according to the invention broken up into its main parts, comprising a suction nozzle (1) with a valve body such as that defined above, in addition to a nasal cannula (9), secretion retaining means (10), support means (11) for supporting the nasal cannula and a flexible antibacterial tube (12).

The nasal suction cannula (9) has a design suitable for being introduced in the nasal passages of an infant, respecting therefore the typical anatomical dimensions and characteristics for performing the initial function of collecting, upon aspirating, the contents of the nasal secretions existing in the choanae. This function is atraumatic, effective and safe, since the material used in the manufacture is plastic, in one embodiment crystal polystyrene (preferably with a butadiene load), with mirror level polish and with the inlet hole of the cannula (9) with atraumatic edges, i.e., with a rounded or blunt finish, without burrs or roughness. The cannula (9) continues with a frustoconical body, the outer wall of which is undulated and with a mirror polish. The cylindrical area of the cannula (the one closest to the user) is located after the frustoconical part, without interruption, which cylindrical area internally houses the secretion retaining means (10), such as a foam filter, a sponge or another suitable element. In the end part opposite the inlet hole there is a region suitable for housing the distal end of support means (11) for supporting the nasal cannula which will hermetically close with the nasal cannula (9), preventing the exit of air through the joint.

In the embodiment exemplified in the drawings, the secretion retaining means (10) consist of a cylindrical sponge having the same radius as the inside of the nasal cannula (9), placed inside the cylindrical part of the nasal cannula and in contact with the support means (10) for supporting the cannula, which serve as an anchor for it. The cavities of the sponge are designed for a high retention of secretions and for facilitating suction.

The support means (11) for supporting the cannula are connected to the nasal cannula (9) at one end and to a flexible antibacterial tube (12) at the other end and the function thereof is to guide and control with the user's fingers the operation of placing and maintaining the position of the cannula (9) in the nasal passages.

The support means (11) furthermore perform a function of deposition of the aspirated secretions when the latter surpass the retaining means (10) due to their abundance.

In the embodiment of Figure 9, a portion of the distal end (approximately 1/5) of the support means (11) is hermetically introduced in the cylindrical part of the nasal cannula (9) until abutting against the secretion retaining means (10) housed inside the cannula, thus fixing said secretion retaining means (10) to prevent the shifting thereof when the suction is performed.

The proximal end of the support means (11) is finished in a small cylindrical tube suitable for being hermetically introduced in the flexible antibacterial tube (12).

The support means (11) for supporting the cannula can be separated with a simple force both from the cannula and from the flexible antibacterial tube (12), for the cleaning and hygiene thereof and for again being placed in the usual prior position thereof after these operations, conserving the original tightness.

In one embodiment, the support means (11) are completely manufactured from opaque materials, for example sieved polypropylene or high-density polyethylene, such that the removed secretions are not visible. The outer walls of the support means (11) have impressions for fingers for the user's comfort and to facilitate the use thereof in terms of a better holding and retention.

The tube (12) forms the mobile part of the assembly joining the suction nozzle (1) with the support means (11) for supporting the cannula. It is manufactured from flexible plastic material with antibacterial properties, preventing the growth of pathogenic germs in its inner and outer walls. The ends of the tube (12) must be perfectly fitted to the suction nozzle (1) and to the support means (11) for supporting the cannula, the tube (12) being able to be separated from both of them for washing or hygiene. In one embodiment, the cylindrical tube is approximately 20 cm long and 0.35 cm wide.

The assembly described above works by the adaptation of the nasal cannula (9) to the nose, placed in the suitable position by means of the support means (11) for supporting the cannula, held and guided by the user, and transmits the suction circulating through the antibacterial tube from the suction nozzle (1) in contact with the user's mouth. The suction of the user opens the flow selection valve system, enabling the suction, but when air is blown or enters the nozzle in the opposite direction, the valve system is closed, preventing a possible transmission of pathogenic germs. The secretions aspirated from the nasal passage accumulate during the process in the secretion retaining means (10). After using the nasal aspirator (8), the nasal cannula (9), the support means (11) for supporting the cannula, the flexible tube (12) and the suction nozzle (1) can be disassembled to clean them and be subsequently assembled again.

## Claims

1. A suction nozzle (1) with a one-way air flow selection valve system for a nasal aspirator, **characterized in that** it comprises:
(a) an inner case (4, 4') containing a suction cannula (5, 5') projecting from its proximal face;
(b) at least one support stop (6, 6') made in the inner part of the suction nozzle (1), which serves as a support for the bending of a valve membrane (7, 7');
(c) a valve membrane (7, 7') supported on the at least one support stop (6, 6') which, when the user sucks in, bends over said stop (6, 6') and allows the air flow towards the suction cannula (5, 5') and which, when the user blows on it, closes the air outlet hole, preventing the flow in the opposite direction; and
(d) an outer case (2, 2'), covering the valve system, except the face from which the suction cannula (5, 5') projects, and containing on its opposite face a hollow coupling body (3, 3') for coupling the suction nozzle (1) to a suitable nasal aspirator.

2. The suction nozzle (1) with a one-way air flow selection valve system according to claim 1, **characterized in that** the valve membrane (7, 7') is housed inside the outer case (2, 2') between the distal face and the support stop (6, 6').

3. The suction nozzle (1) with a one-way air flow selection valve system according to claim 1 or 2, **characterized in that**:
(a) the support stop (6) is made in the inner part of the inner cylindrical case (4) and is formed by four flanges with an end part finished in a point or acute angle;
(b) the coupling body (3) is made in the central area of the distal face of the outer cylindrical case (2).

4. The suction nozzle (1) with a one-way air flow selection valve system according to claim 1 or 2, **characterized in that**:
(a) it comprises two support stops (6') made in the inner part of the outer cylindrical case (2'), which project from the side face, at a distance from the distal face and located facing one another in the lower part of the middle third;
(b) the coupling body (3') is made in the bottom third of the distal face of the outer cylindrical case (2');
(c) the inner case (4') has a step at its distal end at the height of the two support stops (6') of the outer cylindrical case (2') for embedding the elements of the nozzle (1).

5. The suction nozzle (1) with a one-way air flow selection valve system according to claim 4, **characterized in that** the valve membrane (7') has a semicircular reinforcement on the surface which is above the line of the support stops (6').

6. The suction nozzle (1) with a one-way air flow selection valve system according to any of the previous claims, **characterized in that** the inner case (4, 4') and the outer case (2, 2') are cylindrical.

7. A nasal aspirator (8) with a device non inducing contamination and transmission of pathogenic germs, **characterized in that** it comprises:
(a) a nasal cannula (9) containing secretion retaining means (10) therein;
(b) support means (11) for supporting the nasal cannula (9), coupled at one end to the nasal cannula (9), so that the user controls and maintains with his fingers the position of the cannula (9) in the nasal passages;
(c) a tube (12) coupled to a second end of the support means (11);
(d) a suction nozzle (1) with a one-way flow selection valve system according to one of claims 1 to 6 coupled to the free end of the tube (12).

8. The nasal aspirator (8) with a device non inducing contamination and transmission of pathogenic germs according to claim 7, **characterized in that** the nasal cannula (9) has in the inlet holes atraumatic edges, with a rounded or blunt finish.

9. The nasal aspirator (8) with a device non inducing contamination and transmission of pathogenic germs according to claim 7 or 8, **characterized in that** a portion of the support means (11) for supporting the nasal cannula (9) is hermetically introduced in the cylindrical part of the nasal cannula, fixing the secretion retaining means (10) to prevent the shifting thereof during the suction.

10. The nasal aspirator (8) with a device non inducing contamination and transmission of pathogenic germs according to any of claims 7 to 9, **characterized in that** the tube (12) joining the support means (11) for supporting the nasal cannula (9) to the suction nozzle (1) with a valve system is flexible and is manufactured in plastic with properties preventing bacterial growth.
